# EUROPEAN PATENT APPLICATION

(11) **EP 2 902 009 A1**
(43) Date of publication of application: **05.08.2015**
(21) Application number: 13841696.1
(22) Date of filing: 20.08.2013
(51) Int. Cl.: A61K 8/37, A61K 8/34, A61Q 19/00

(54) **COSMETIC COMPOSITION, CONTAINING XYLITOL FATTY ACID ESTERS AS ACTIVE INGREDIENT, HAVING CONTINUOUS COOLING EFFECT**

(30) Priority: 25.09.2012 KR 20120106717
(71) Applicant: Chi, Jun-Hong, Yongin-si, Gyeonggi-do 446-788 (KR)
(72) Inventor: BAE, Dae Ho, Hwaseong-si Gyeonggi-do 445-995 (KR); CHOI, Suk In, Anyang-si Gyeonggi-do 431-061 (KR)
(74) Representative: Viering, Jentschura & Partner Patent- und Rechtsanwälte
(86) International application number: PCT/KR2013/007470
(87) International publication number: WO 2014/051259

(57) **Abstract**

The present invention relates to a cosmetic composition containing xylitol fatty acid esters, and more particularly to a cooling sensation agent for a cosmetic composition containing xylitol fatty acid esters having emulsion stability and continuous cooling sensation effect as an active ingredient. The cosmetic composition containing xylitol fatty acid esters according to the present invention can have a continuous cooling sensation effect for a longer time as compared to the conventional xylitol-containing cosmetic compositions. Furthermore, along with the continuous cooling effect, smooth spreadability and high absorbability of a cosmetic agent can be seen when the xylitol fatty acid esters of the present invention is used instead of the xylitol which may cause the problems of the spreadability, absorbability and emulsion stability thereof.

## Description

### [Technical Field]

The present invention relates to a cosmetic composition including a xylitol fatty acid ester. More particularly, the present invention relates to a cooling sensation agent for a cosmetic composition including a xylitol fatty acid ester having emulsion stability and continuous cooling sensation effect as an active ingredient.

### [Background Art]

A general cosmetic composition is an assembly of materials formed into a type of emulsion by introducing an emulsifier to aqueous ingredients, such as water and glycerin, and oily ingredients, such as oil, wax and butter. In addition, in the case of recent cosmetic compositions, functional materials, such as moisturizing, whitening and UV protecting agents are added to such basic materials.

One example of such functional materials that are used widely is a cooling sensation agent including a heat-absorbing material, such as xylitol, erythritol or menthol, added to reduce the skin temperature increasing in hot seasons, such as summer.

Xylitol is a plant-derived material and is obtained by subjecting xylose present in birch or corn to hydrogenation. More particularly, methods for preparing xylitol generally include reducing xylose obtained by hydrolyzing xylan contained in vegetable materials in the presence of a catalyst to convert it into xylitol, or preparing xylitol by means of microbials. For example, xylitol may be obtained from glucose by using yeast. In the case of such a method for preparing xylitol from glucose by means of microbials, it is possible to obtain xylitol by using no severe conditions and dangerous processes, such as hydrolysis of xylan contained in vegetable materials or hydrogenation of xylose using a metal catalyst, which leads to a decrease in cost required for xylitol preparation.

Xylitol is a sugar alcohol material having five carbon atoms (C₅H₁₂O) and a molecular weight of 152.15 and is a low-calorie material. Xylitol also has anticariogenic activity, since it inhibits the glycolysis action of Streptococcus mutans, one of cariogenic bacteria, and inactivates it, reduces formation of plaque, a bacterial membrane on the surface of a tooth, and reduces formation of acids in plaque. Thus, xylitol is used in chewing gum, cookies, candies, or the like.

Referring to the patents and conventional cosmetic agents using xylitol, xylitol has been used in a small proportion of pack compositions or skin application cosmetic agents based on the fact that xylitol causes endothermic reaction when it is dissolved into water. In addition, in various domestic and foreign patents, there has been suggested that sugar alcohols including xylitol have a heat of dissolution of - kcal/kg (negative value) and show a cooling sensation function.

However, in the case of sugar alcohols such as xylitol, particularly in the case of xylitol, although it causes endothermic reaction when it is dissolved into water and absorbs heat, it is sugar in itself, and thus has a significant disadvantage despite its functionality in that it provides an undesirable sticky feel after dissolution. Therefore, use of xylitol in mask products such as pack is somewhat problematic. Particularly, when using xylitol in skin application cosmetic products requiring smooth spreadability and absorbability, xylitol realizes a cooling sensation function but inhibits absorbability due to its sugar-based characteristics including stickiness and hard film forming property. This makes it difficult to use xylitol in cosmetic compositions such as cream or lotion, resulting in a significant problem.

Although xylitol may be used alone for the only purpose of realizing a cooling sensation function, it is not proper to use xylitol in spite of degradation of the fundamental properties of a cosmetic agent, including spreadability and absorbability. Particularly, in summer requiring a cooling sensation function, the use of a cosmetic agent containing xylitol may be rejected when it shows stickiness and poor absorbability.

It is certain that xylitol is one of the heat-absorbing materials such as sugar alcohols and has an excellent cooling sensation effect and solubility, and thus it is a good material as a cooling sensation agent. However, it is not preferred to use xylitol in spite of degradation of the fundamental properties of a cosmetic agent, including spreadability and absorbability.

Under these circumstances, the present inventors have conducted many studies to provide a xylitol ester material by processing xylitol so that the disadvantage of xylitol, i.e., stickiness unique to sugar itself may be improved, the problem of deemulsification caused by excessive introduction of xylitol may be solved, and a cooling sensation effect may be maintained for a longer time as compared to conventional xylitol.

### [Disclosure]

### [Technical Problem]

A technical problem to be solved by the present invention is to provide a cosmetic composition capable of maintaining a cooling sensation effect and including, as an active ingredient, a xylitol fatty acid ester having improved emulsion stability and a continuous cooling sensation effect as compared to the conventional xylitol.

### [Technical Solution]

In one general aspect, there is provided a cooling sensation agent for cosmetic composition including a xylitol fatty acid ester having a continuous cooling sensation effect as an active ingredient.

As used herein, 'cooling sensation agent' means a material that reduces the skin temperature upon the application to the skin and imparts a cooling sensation. Typical materials that added to the conventional cosmetic compositions for providing a cooling sensation effect are sugar alcohols. Such sugar alcohols include xylitol, erythritol, sorbitol and mannitol, particularly xylitol. However, most of such sugar alcohols, except xylitol, merely impart a sensation of refreshment not a cooling sensation effect derived from an actual drop of skin temperature. Unlike xylitol, when such sugar alcohols are used in a large amount, they cause skin irritation, and thus are limited in application to the face.

In the case of xylitol, it has stickiness and is viscous. In addition, when using a large amount of xylitol, it breaks the water/oil phase emulsion formulation of a cosmetic agent and makes the emulsion state unstable. Therefore, when using xylitol in a cosmetic agent, there is a difficulty in that attentions should be given to the amount of use and control of water/oil phase emulsification.

The present invention is intended to solve the problems of stickiness and degraded absorbability of xylitol while maintaining the cooling sensation effect of xylitol itself by preparing a xylitol fatty acid ester through the esterification of xylitol with a fatty acid in order to improve such problems caused by the properties unique to xylitol.

Xylitol used herein is a five-carbon sugar alcohol and has a structure represented by the following Chemical Formula 1.

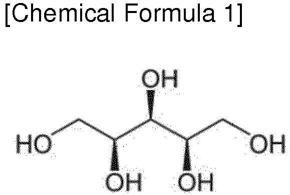

In addition, the fatty acid that may be used herein include caprylic acid, capric acid, lauric acid, myristic acid, palmitic acid, oleic acid, olive oil fatty acid, erucylic acid and behenic acid, caprylic acid, lauric acid and olive oil fatty acid being preferred. Such fatty acids show more stable and effective cooling sensation action upon the application to cosmetic agents, and thus induce smooth spreadability and high absorbability.

Particularly, caprylic acid, lauric acid and olive oil fatty acid may be useful. When it is most important to realize a cooling sensation effect, a fatty acid having a low carbon number, such as caprylic acid, may be used. As the carbon number of fatty acid increases, the cooling sensation effect slightly decreases but the spreadability and absorbability are improved. Thus, in this case, it is possible to obtain improved long-term cooling sensation effect as compared to xylitol.

In the cosmetic composition according to the present invention, the xylitol fatty acid ester may be xylitol caprylate, xylitol laurate or xylitol olive oil fatty acid ester.

The xylitol fatty acid ester may be selected depending on the intended use. For example, when it is most important to realize a cooling sensation effect, xylitol caprylate is suitable. When it is important to realize high spreadability and absorbability while maintaining a cooling sensation effect, a xylitol olive oil fatty acid ester is suitable. In addition, xylitol laurate is in the middle between xylitol caprylate and xylitol olive oil fatty acid ester.

In the cosmetic composition according to the present invention, there is no limitation in the mixing ratio and proportion of a xylitol fatty acid ester. However, since the xylitol fatty acid ester is a material having properties as emulsifier, the following ratio may be used preferably. It is preferred to introduce the xylitol fatty acid ester in an amount of 0.1-10 wt% based on the total weight of cosmetic composition. It is more preferred to introduce 2-5 wt% of xylitol fatty acid ester in order to realize a cooling sensation effect, induce emulsion stability and to provide smooth spreadability and high absorbability. However, the xylitol fatty acid ester may be used in an amount varying with the particular use.

The cosmetic composition according to the present invention is characterized in that it has a higher duration of cooling sensation effect as compared to the conventional xylitol-containing cosmetic compositions. As used herein, a continuous cooling sensation effect means that the cosmetic composition maintains an effect of cooling body temperature for at least 4 minutes after applying the cosmetic composition to the skin.

The duration of cooling sensation effect may be determined as compared to the conventional xylitol-containing cosmetic compositions. According to an embodiment, when using a xylitol fatty acid ester in a cosmetic composition, the cooling sensation effect shows a higher duration as compared to the cooling sensation effect of xylitol. Particularly, after evaluating the cooling sensation effect, it is demonstrated that the use of xylitol itself provides high cooling sensation effect instantly upon the skin application based on the normal body temperature, but the body temperature returns to the normal body temperature 3 minutes after the completion of the skin application. On the contrary, in the case of the xylitol fatty acid ester according to the present invention, the instant body temperature cooling effect is slightly low but the cooling sensation effect is maintained for at least 5 minutes to 6 minutes. Thus, the xylitol fatty acid ester shows a duration of cooling sensation at least twice higher than xylitol (see Example 4).

The cosmetic composition according to the present invention is characterized in that it has higher emulsion stability as compared to the conventional xylitol-containing cosmetic compositions.

According to an embodiment, cosmetic samples are prepared by using each of xylitol and xylitol fatty acid ester and the emulsion state of each sample is determined. In the case of a xylitol-containing sample, it is observed by the naked eyes that precipitation and deemulsification occur after the lapse of a certain time. On the contrary, a xylitol ester-containing sample causes no deemulsification and shows a more stable emulsion state (see Example 3).

Thus, although xylitol itself has a high cooling sensation effect, it may adversely affect the emulsion stability after being formulated into a cosmetic composition. Therefore, xylitol has a disadvantage in that it is limited in amount of use. However, the xylitol ester according to the present invention itself may be used as emulsifier or supplementary emulsifier, and thus is not limited in amount of use. However, since no emulsifier or supplementary emulsifier is used in an amount of several tens wt%, it is preferred to use the xylitol ester in the above-defined range.

The cosmetic composition including a xylitol fatty acid ester according to the present invention is not limited to any particular formulation. The cosmetic composition according to the present invention may have a formulation of skin lotion, skin softener, skin toner, astringent, lotion, nourishing lotion, milk lotion, moisturizing lotion, nourishing cream, massage cream, moisturizing cream, hand cream, essence, nourishing essence, pack, soap, cleansing foam, cleansing lotion, cleansing cream, body cleanser, body lotion, shampoo, emulsion, foundation, pressed powder, loose powder, eye shadow, or the like.

### [Advantageous Effects]

According to the embodiments of the present invention, the cosmetic composition including a xylitol fatty acid ester realizes a continuous cooling sensation effect for a longer time as compared to the conventional xylitol-containing cosmetic compositions. In addition, when using the xylitol fatty acid ester according to the present invention instead of xylitol that may cause the problems of the spreadability, absorbability and emulsion stability of a cosmetic agent, it is possible to realize smooth spreadability and high absorbability because the xylitol fatty acid ester is a material having properties as emulsifier in combination with a continuous cooling sensation effect.

### [Description of Drawings]

Fig. 1 is a chart of gas chromatography analysis for the fatty acid composition of olive oil obtained according to an embodiment.
Fig. 2 is a graph showing the cooling sensation effect of each of the cosmetic composition including a fundamental formulation, xylitol-containing composition and xylitol fatty acid ester-containing composition.
Fig. 3 is a graph showing the moisturizing effect of each of the cosmetic composition including a fundamental formulation and xylitol fatty acid ester-containing composition.

### [Best Mode]

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein.

### Materials

Xylitol used herein is Xylisorb-700 available from Roqette Co. (France). Caprylic acid used for preparing a xylitol fatty acid ester is a product commercially available from JUNSEI Co. (min. 98.5%) and lauric acid is a product commercially available from Palm-Oleo Co. Olive oil is one commercially available from B&T Co. (Italy).

### Example 1: Preparation of Olive Oil Fatty Acids

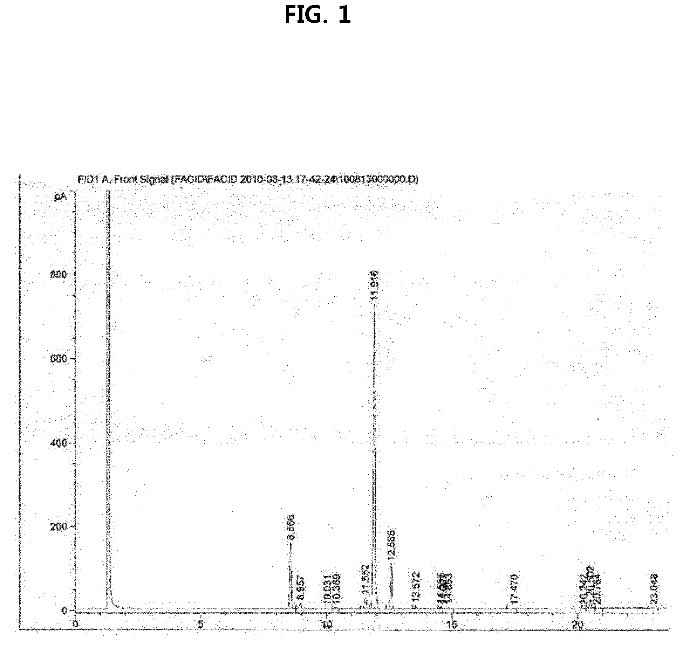

First, olive oil and distilled water are introduced at the same weight proportion and agitated, and then the resultant mixture is introduced to SUS316L reactor resistant against high temperature and high pressure. Then, the mixture is subjected to a high temperature/high pressure process by maintaining the reactor under 20 atm at 180°C for 7 hours. After the completion of reaction, the reaction materials are allowed to stand and the lower layer mixture formed of distilled water and glycerin is discharged out to obtain the upper layer formed of fatty acids. Since the upper layer of fatty acids also includes distilled water and glycerin, it is dehydrated under reduced pressure to remove water completely and subjected to washing with water and dehydration twice or three times to remove the remaining glycerin and water completely. The yield of fatty acids is 95-99%. The thus obtained olive oil fatty acids are analyzed for the composition of fatty acids by gas chromatography and the results are shown in Fig. 1. In addition, Table 1 shows the composition of fatty acids obtained from the olive oil.

**[Table 1]**

| Composition of olive oil fatty acids after hydrolysis | |
|---|---|
| Palmitic acid | 10.0 |
| Palmitoleic acid | 0.5 |
| Stearic acid | 3.1 |
| Oleic acid | 74 |
| Linoleic acid | 10.9 |
| Linolenic acid | 0.5 |
| Others | 1 |
| Total | 100 |

The composition of olive oil fatty acids that may be used herein is not limited to Table 1. Typical fatty acids forming the olive oil that may be used herein include palmitic acid, palmitoleic acid, stearic acid, oleic acid, linoleic acid and linolenic acid. Olive oil has a different composition of fatty acids depending on its seed. In addition, olive oil has a different composition depending on its hydrolysis method. Thus, the compositional ratio is shown as an approximate ratio.

### Example 2: Preparation of Xylitol Fatty Acid Esters

The olive oil fatty acids obtained from Example 1, caprylic acid and lauric acid are used to carry out esterification of xylitol. In this manner, xylitol fatty acid esters are obtained. The esterification is carried out as follows.

First, xylitol and each of the acids (caprylic acid, lauric acid and olive oil fatty acids obtained from Example 1) are mixed at the same amount (i.e. molar ratio of 1:1), and an acid catalyst (PTSA, para-toluenesulfonic acid, 99%, Daejung Chemicals & Metals Co., Ltd.) is introduced thereto in an amount of 0.2 w/w% based on the weight of fatty acids. Then, the materials are mixed thoroughly while xylitol is dissolved under heating. The mixed materials are subjected to dehydration at 200°C or lower for 8 hours under nitrogen. The water produced through the esterification is separated by way of a cooling condenser and the reaction is allowed to proceed.

The xylitol and fatty acids remaining in crude ester after the completion of reaction are removed by distillation under reduced pressure. When the acid value is at least 1 mgKOH/g or less, distillation under reduced pressure is finished and decolorization/deodorization is carried out by using active carbon and terra alba. In this manner, xylitol caprylate, xylitol laurate and xylitol olive oil fatty acid ester are obtained according to the present invention.

### Example 3: Comparison between Xylitol and Xylitol Ester in Terms

### of Emulsion Stability

Xylitol and xylitol ester are used to provide samples with a fundamental lotion formulation, and a test for emulsion stability is carried out. The lotion formulation used herein is shown in Table 2.

**[Table 2]**

| Materials | Fundamental formulation | Xylitol 2% | Xylitol 4% | Xylitol ester 2% | Xylitol ester 4% |
|---|---|---|---|---|---|
| DI-Water | 54.22 | 52.22 | 50.22 | 52.22 | 50.22 |
| EDTA-2Na | 0.02 | 0.02 | 0.02 | 0.02 | 0.02 |
| Methyl Paraben | 0.20 | 0.20 | 0.20 | 0.20 | 0.20 |
| Glycerin | 2.00 | 2.00 | 2.00 | 2.00 | 2.00 |
| 1,3-BG | 3.00 | 3.00 | 3.00 | 3.00 | 3.00 |
| Xylitol | - | 2.00 | 4.00 | - | - |
| TEA | 0.06 | 0.06 | 0.06 | 0.06 | 0.06 |
| Keltrol F | 0.20 | 0.20 | 0.20 | 0.20 | 0.20 |
| DI-Water | 20.00 | 20.00 | 20.00 | 20.00 | 20.00 |
| Olivem 1000 | 2.00 | 2.00 | 2.00 | 2.00 | 2.00 |
| Xylityl Ester | - | - | - | 2.00 | 4.00 |
| Lanette-O | 0.40 | 0.40 | 0.40 | 0.40 | 0.40 |
| KM-105(GMS) | 0.50 | 0.50 | 0.50 | 0.50 | 0.50 |
| Propyl Paraben | 0.10 | 0.10 | 0.10 | 0.10 | 0.10 |
| KF96(6cs) | 1.00 | 1.00 | 1.00 | 1.00 | 1.00 |
| Squalane | 4.00 | 4.00 | 4.00 | 4.00 | 4.00 |
| Dermofeel BGC | 6.00 | 6.00 | 6.00 | 6.00 | 6.00 |
| Carbopol-941 1% Sol. | 6.00 | 6.00 | 6.00 | 6.00 | 6.00 |
| Phenoxyethanol | 0.30 | 0.30 | 0.30 | 0.30 | 0.30 |
| Total | 100.00 | 100.00 | 100.00 | 100.00 | 100.00 |
| pH | 6.80 | 6.82 | 6.83 | 6.76 | 6.80 |

Each of the samples is prepared based on the formulations as shown in Table 2 and stored in a constant temperature chamber to check the emulsion state of each sample every day. It is shown by the naked eyes that the lotion sample containing 2% xylitol causes separation of precipitation and deemulsification after 1 month, and the lotion sample containing 4% xylitol causes separation and deemulsification after 3 weeks. However, xylitol caprylate causes no deemulsification like the conventional emulsifier for use in cosmetics and shows a stable emulsion state. Therefore, it is expected that a xylitol ester having properties as a stable emulsifier provides smooth spreadability and absorbability to cosmetics.

### Example 4: Comparison between Xylitol and Xylitol Fatty Acid Ester in Terms of Cooling Sensation Effect

Xylitol is differentiated clearly from a xylitol fatty acid ester in terms of properties, and thus shows a cooling sensation effect, spreadability and absorbability different from those of xylitol. First, xylitol and a xylitol fatty acid ester are compared with each other in terms of cooling sensation effect.

Each of the samples is prepared according to the fundamental lotion formulation as shown in Table 2. Then, ten practicians of cosmetic industry are allowed to participate in this test as subjects, the body temperature of each subject is measured at his/her left hand, each sample is applied to the hand in an amount of 1 g per run for 30 seconds, the body temperature of each subject is measured again, and the variations in body temperature are determined at an interval of 1 minute. Measurement of the body temperature is carried out by using a skin IR clinical thermometer, ThermoFlas LX-26 imported by and available from BORUNG Soo & Soo Ltd. The thermometer is maintained at a distance of 3 cm from the skin. In addition, when determining each sample, the first sample is made by the fundamental formulation. Once the same body temperature as the temperature measured in the case of the fundamental formulation is restored after waiting, the sample is applied again and the body temperature is measured again.

The results are shown in Table 3. The results are shown as averages, and the conventional fundamental formulation and formulations using 2% xylitol, 2% xylitol caprylate, 2% xylitol laurate, and 2% xylitol olive oil fatty acid ester are included in the test list.

**[Table 3]**

| Body temperature measurement (°C) | Fundamental formulation | Xylitol | Xylitol caprylate | Xylitol laurate | Xylitol olive oil fatty acid ester |
|---|---|---|---|---|---|
| Before application | 32.6 | 32.6 | 32.6 | 32.6 | 32.6 |
| After application | 31.8 | 31.3 | 31.0 | 31.0 | 31.4 |
| After 1 min. | 32.5 | 30.9 | 31.2 | 31.0 | 31.4 |
| After 2 min. | 32.6 | 32.0 | 32.0 | 31.8 | 31.5 |
| After 3 min. | - | 32.6 | 32.1 | 32.0 | 31.8 |
| After 4 min. | - | - | 32.4 | 32.3 | 32.1 |
| After 5 min. | - | - | 32.6 | 32.5 | 32.4 |
| After 6 min. | - | - | - | - | 32.6 |

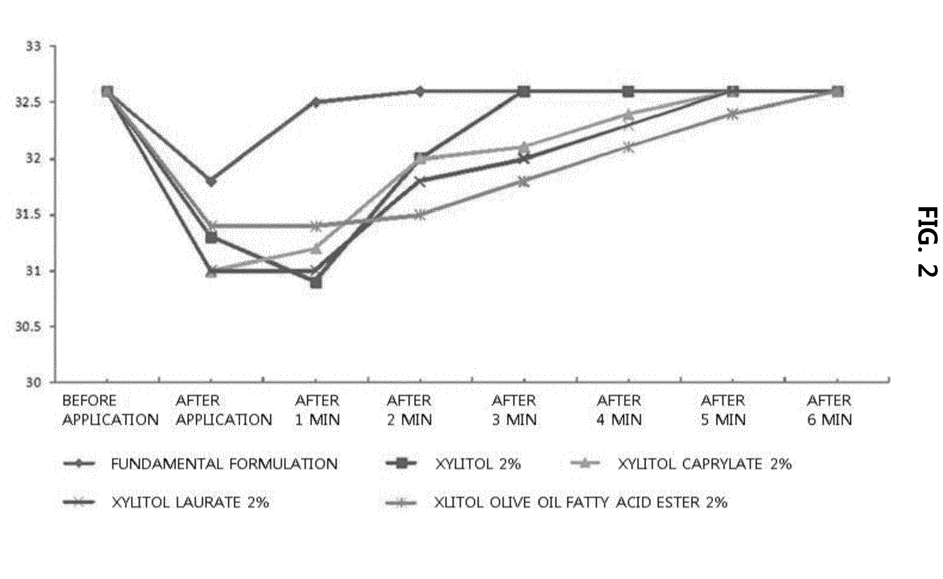

The results of Table 3 are plotted in Fig. 2. It can be seen from the results that when using 2% xylitol alone, there is provided a high instant cooling sensation effect corresponding to -1.7°C Vs. the original body temperature but the body temperature is restored to the original temperature 3 minutes after the completion of application. On the contrary, in the case of a xylitol fatty acid ester, the instant body temperature cooling sensation effect is slightly low but the effect is maintained for at least 5 minutes to 6 minutes. This demonstrates that a xylitol fatty acid ester shows a duration of cooling sensation effect at most twice higher as compared to xylitol.

### Example 5: Test for Moisturizing Effect of Xylitol Fatty Acid Ester

To determine the moisturizing effect of xylitol fatty acid ester according to the present invention, xylitol olive oil fatty acid ester is evaluated and compared with the fundamental formulation by using Corneometer CM820, Tewameter TM210 available from CK Electronic Co. (Germany).

Preparation is made before the evaluation of moisturizing effect as follows. To evaluate the skin moisture capacity derived from lotion, ten subjects at the age of 20-30 are selected, stabilized under a constant temperature constant humidity condition (25-30°C, relative humidity 40-60%) for a predetermined time (at least 1 hour), and made to know precautions. Then, test consents are made by the subjects, and the skin moisture capacity is determined right after applying each sample and 180 minutes after applying each sample.

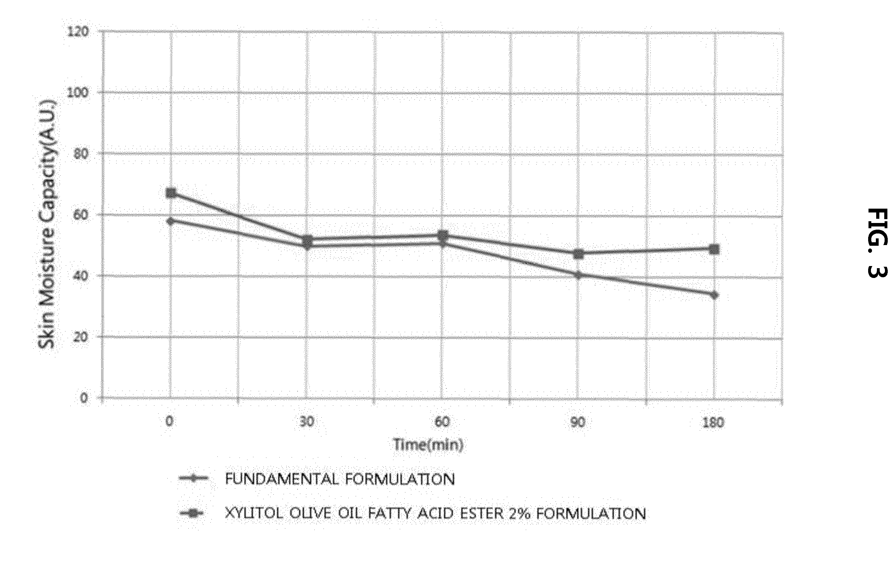

The test is carried out as follows. Lotion is applied to the inside of each subject's arm and moisture capacity is measured. The measurement of moisture capacity is carried out by using Corneometer CM820 five times. Then, the measured values are converted into arbitrary capacitance units (A.U.) between 0 and 120. All tests are repeated three times and the statistical analysis is performed by Student's t-test at a significance level of 5%. The results of test for moisturizing effect are calculated by averaging the results of 10 subjects. The results are shown in the graph of Fig. 3.

As shown in Fig. 3, the formulation including 2% xylitol olive oil fatty acid ester shows relatively higher moisturizing effect. The results are shown in Table 4 in detail.

**[Table 4]**

| | Elapse Time after Application | | | | |
|---|---|---|---|---|---|
| | Initial | 30 min. | 60 min. | 90 min. | 180 min. |
| Fundamental Formulation | 58.25 | 50 | 51 | 41 | 34.5 |
| Xylitol ester | 67.25 | 52.25 | 53.75 | 47.75 | 49.5 |

It can be seen from the above test results that the cosmetic composition using a xylitol fatty acid ester according to the present invention provides a slightly lower cooling sensation effect at the initial stage but realizes long-term cooling sensation effect at most approximately twice higher as compared to the conventional cosmetic composition using xylitol. In addition, when using a xylitol fatty acid ester instead of xylitol that may cause a problem of emulsion stability in a cosmetic formulation, it is possible to improve the formulation stability and to provide high moisturizing effect.

## Claims

1. A cooling sensation agent for cosmetic composition comprising a xylitol fatty acid ester having a continuous cooling sensation effect as an active ingredient.

2. The cooling sensation agent for cosmetic composition according to claim 1, wherein the xylitol fatty acid ester is xylitol caprylate, xylitol laurate or xylitol olive oil fatty acid ester.

3. The cooling sensation agent for cosmetic composition according to claim 1, wherein the xylitol fatty acid ester is used in an amount of 0.1-10 wt% based on the total weight of composition.

4. The cooling sensation agent for cosmetic composition according to claim 1, wherein the composition provides a higher duration of cooling sensation effect as compared to a xylitol-containing cosmetic composition.

5. The cooling sensation agent for cosmetic composition according to claim 1, wherein the composition provides higher emulsion stability as compared to a xylitol-containing cosmetic composition.

6. The cooling sensation agent for cosmetic composition according to claim 1, wherein the composition has a formulation selected from the group consisting of skin lotion, skin softener, skin toner, astringent, lotion, nourishing lotion, milk lotion, moisturizing lotion, nourishing cream, massage cream, moisturizing cream, hand cream, essence, nourishing essence, pack, soap, cleansing foam, cleansing lotion, cleansing cream, body cleanser, body lotion, shampoo, emulsion, foundation, pressed powder, loose powder, and eye shadow.
